# EUROPÄISCHE PATENTSCHRIFT

(11) **EP 4 374 818 B1**
(45) Veröffentlichungstag und Bekanntmachung des Hinweises auf die Patenterteilung: **09.04.2025**
(21) Anmeldenummer: 22209984.8
(22) Anmeldetag: 28.11.2022
(51) Int. Cl.: A61C 13/00, B33Y 80/00

(54) **VERFAHREN ZUM HERSTELLEN EINER DENTALEN RESTAURATION**
METHOD FOR PRODUCING A DENTAL RESTORATION
PROCÉDÉ DE FABRICATION D'UNE RESTAURATION DENTAIRE

(43) Veröffentlichungstag der Anmeldung: 29.05.2024
(73) Patentinhaber: Ivoclar Vivadent AG, 9494 Schaan (LI)
(72) Erfinder: Atilim, Eser, 88131 Lindau (DE); Hendrik, John, 9470 Buchs (CH)
(74) Vertreter: Baldus, Oliver

(56) Entgegenhaltungen:
- US-A1- 2011 276 159
- US-A1- 2018 333 235

## Beschreibung

Die vorliegende Erfindung betrifft ein Verfahren zum Herstellen einer dentalen Restauration und eine Herstellungsvorrichtung zum Herstellen einer dentalen Restauration.

Die gegenwärtige Herstellung von Prothesen ist ein arbeitsintensiver Prozess. Besonders die Optimierung der Prothesengeometrie, um den größtmöglichen Tragekomfort zu erreichen, erfordert oft zahlreiche Arztbesuche. US 2018/333235 A1 beschreibt ein Verfahren zum digitalen Entwerfen von Zahnprothesen unter Modellierung eines Verteilungsmodells für wunde Stellen, an denen ein Patient aufgrund von Druck, der auf die Mundschleimhaut ausgeübt wird, Schmerzen empfindet, wenn der Patient die Zahnprothese trägt.

Es ist die technische Aufgabe der vorliegenden Erfindung, den Tragekomfort einer dentalen Restauration zu erhöhen und Schmerzen und Beschwerden beim Tragen der dentalen Restauration zu vermindern.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration gelöst, mit den Schritten eines Berechnens einer Druckverteilung auf die Mundschleimhaut durch die dentale Restauration auf Basis eines dreidimensionalen Datensatzes der dentalen Restauration und eines dreidimensionalen Datensatzes der Mundschleimhaut; und eines Bestimmens eines räumlichen Bereiches für ein elastisches Herstellungsmaterial innerhalb der dentalen Restauration auf Basis der berechneten Druckverteilung. Der räumliche Bereich bildet ein Untervolumen der dentalen Restauration. Durch das Verfahren wird der technische Vorteil erreicht, dass der Tragekomfort der dentalen Restauration verbessert wird. Zudem kann eine langfristige Restkammresorption der dentalen Restauration verhindert werden.

In einer technisch vorteilhaften Ausführungsform des Verfahrens wird ein Herstellungsmaterial für den räumlichen Bereich auf Basis der Druckverteilung bestimmt. Das Herstellungsmaterial kann aus einer Mehrzahl von Herstellungsmaterialien ausgewählt werden. Dadurch wird beispielsweise der technische Vorteil erreicht, dass nicht nur eine Ausdehnung des räumlichen Bereichs, sondern auch ein Herstellungsmaterial auf Basis der Druckverteilung festgelegt werden kann. Dadurch lässt sich der Tragekomfort der dentalen Restauration nochmals verbessern.

In einer weiteren technisch vorteilhaften Ausführungsform des Verfahrens wird die Druckverteilung mittels einer Finite-Elemente-Methode berechnet. Dadurch wird beispielsweise der technische Vorteil erreicht, dass sich die Druckverteilung auf schnelle Weise mit hoher Genauigkeit berechnen lässt.

In einer weiteren technisch vorteilhaften Ausführungsform des Verfahrens wird die Druckverteilung bei Vorliegen einer vorgegebenen Kraft auf die dentale Restauration berechnet. Dadurch wird beispielsweise der technische Vorteil erreicht, dass auftretende Kräfte bei der Berechnung der Druckverteilung berücksichtigt werden können und bei der Gestaltung des räumlichen Bereichs berücksichtigt werden.

In einer weiteren technisch vorteilhaften Ausführungsform des Verfahrens wird die vorgegebene Kraft durch einen Aufbiss-Sensor ermittelt. Dadurch wird beispielsweise der technische Vorteil erreicht, dass realistische Kräfte und Angriffspunkte bei der Berechnung der Druckverteilung verwendet werden.

In einer weiteren technisch vorteilhaften Ausführungsform des Verfahrens wird aus der Druckverteilung auf die Mundschleimhaut eine Druckverteilung auf die dentale Restauration berechnet. Dadurch wird beispielsweise der technische Vorteil erreicht, dass sich der räumliche Bereich innerhalb der dentalen Restauration genau anpassen lässt.

In einer weiteren technisch vorteilhaften Ausführungsform des Verfahrens ist eine Dicke des räumlichen Bereichs proportional zur Druckverteilung auf die dentale Restauration. Dadurch wird beispielsweise der technische Vorteil erreicht, dass sich der räumliche Bereich auf einfache Weise berechnen lässt.

In einer weiteren technisch vorteilhaften Ausführungsform des Verfahrens weist das Herstellungsmaterial in dem räumlichen Bereich ein niedrigeres Elastizitätsmodul als das Herstellungsmaterial außerhalb des räumlichen Bereichs auf. Dadurch wird beispielsweise der technische Vorteil erreicht, dass der Tragekomfort der dentalen Restauration gesteigert werden kann.

In einer weiteren technisch vorteilhaften Ausführungsform des Verfahrens wird das Herstellen der dentalen Restauration mittels eines dreidimensionalen Druckverfahrens durchgeführt. Dadurch wird beispielsweise der technische Vorteil erreicht, dass sich die dentale Restauration auf eine einfache Weise herstellen lässt.

In einer weiteren technisch vorteilhaften Ausführungsform des Verfahrens verwendet das dreidimensionale Druckverfahren einen Freistrahl-Materialauftrag. Dadurch wird beispielsweise der technische Vorteil erreicht, dass die Herstellung der dentalen Restauration nochmals verbessert wird.

Gemäß einem zweiten Aspekt wird die technische Aufgabe durch ein eine Herstellungsvorrichtung zum Herstellen einer dentalen Restauration gelöst, mit einer Berechnungsvorrichtung zum Berechnen einer Druckverteilung auf die Mundschleimhaut durch die dentale Restauration auf Basis eines dreidimensionalen Datensatzes der dentalen Restauration und eines dreidimensionalen Datensatzes der Mundschleimhaut; und einer Bestimmungsvorrichtung zum Bestimmen eines räumlichen Bereiches für ein elastisches Herstellungsmaterial innerhalb der dentalen Restauration auf Basis der berechneten Druckverteilung.

In einer technisch vorteilhaften Ausführungsform der Herstellungsvorrichtung ist die Berechnungsvorrichtung ausgebildet, die Druckverteilung mittels einer Finite-Elemente-Methode zu berechnen. Dadurch wird beispielsweise ebenfalls der technische Vorteil erreicht, dass sich die Druckverteilung auf schnelle Weise mit hoher Genauigkeit berechnen lässt.

In einer weiteren technisch vorteilhaften Ausführungsform der Herstellungsvorrichtung ist die Bestimmungsvorrichtung ausgebildet, eine Dicke des räumlichen Bereichs proportional zur Druckverteilung auf eine dentale Restauration zu bestimmen.

In einer weiteren technisch vorteilhaften Ausführungsform der Herstellungsvorrichtung ist die Herstellungsvorrichtung ausgebildet, innerhalb des räumlichen Bereiches ein Herstellungsmaterial mit einem niedrigeren Elastizitätsmodul als außerhalb des räumlichen Bereichs zu verwenden.

In einer weiteren technisch vorteilhaften Ausführungsform der Herstellungsvorrichtung umfasst das Herstellungsgerät einen 3D-Drucker. Dadurch wird beispielsweise der technische Vorteil erreicht, dass sich die dentale Restauration auf einfache Weise herstellen lässt.

Ausführungsbeispiele der Erfindung sind in den Zeichnungen dargestellt und werden im Folgenden näher beschrieben.

Es zeigen:
- Fig. 1: eine perspektivische Ansicht einer dentalen Restauration innerhalb eines Mundraums;
- Fig. 2: eine Druckverteilung auf einer Mundschleimhaut;
- Fig. 3: eine Querschnittsansicht durch die dentale Restauration und die Mundschleimhaut;
- Fig. 4: eine weitere Querschnittsansicht durch die dentale Restauration und die Mundschleimhaut;

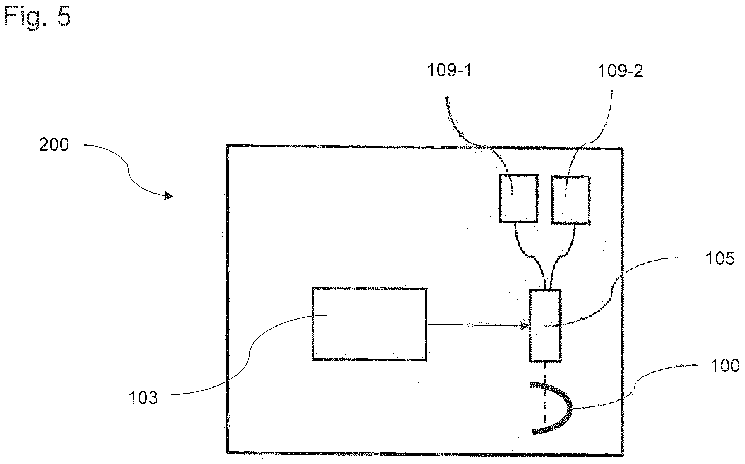

- Fig. 5: eine schematische Ansicht einer Herstellungsvorrichtung zum Herstellen einer dentalen Restauration; und
- Fig. 6: ein Blockdiagramm eines Verfahrens zum Herstellen der dentalen Restauration.

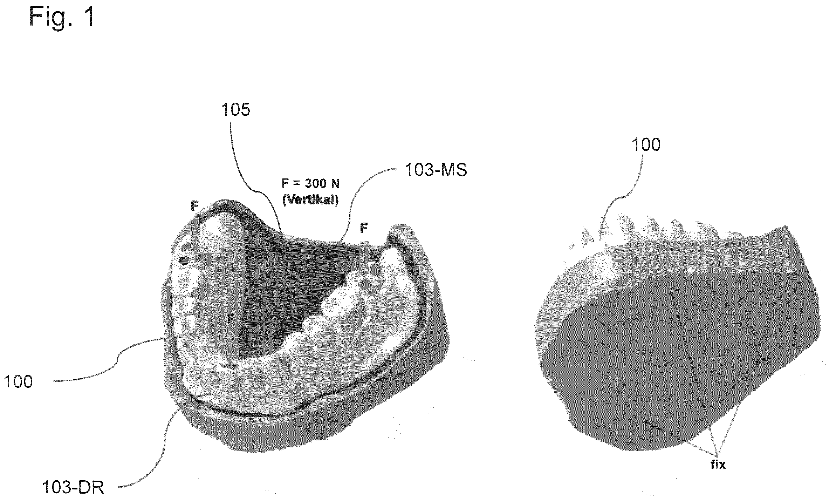

Fig. 1 zeigt eine perspektivische Ansicht einer dentalen Restauration 100 innerhalb eines Mundraums. Die dentale Restauration 100 ist beispielsweise eine Teil- oder Vollprothese, die zumindest teilweise auf einer Mundschleimhaut 105 aufsitzt und mit dieser in Kontakt steht.

Die Form der dentalen Restauration 100 ist in einem dreidimensionalen Datensatz 103-DR angegeben. Dieser kann zusätzlich Farbdaten oder Daten über das zu verwendende Herstellungsmaterial der Dentalen Restauration 100 umfassen. Die Form der Mundschleimhaut 105 des Patienten ist ebenfalls in einem dreidimensionalen Datensatz 103-MS gespeichert. Auf die dentale Restauration werden beim Tragen unterschiedliche Kräfte F ausgeübt, wie beispielsweis beim Kauen von Nahrung.

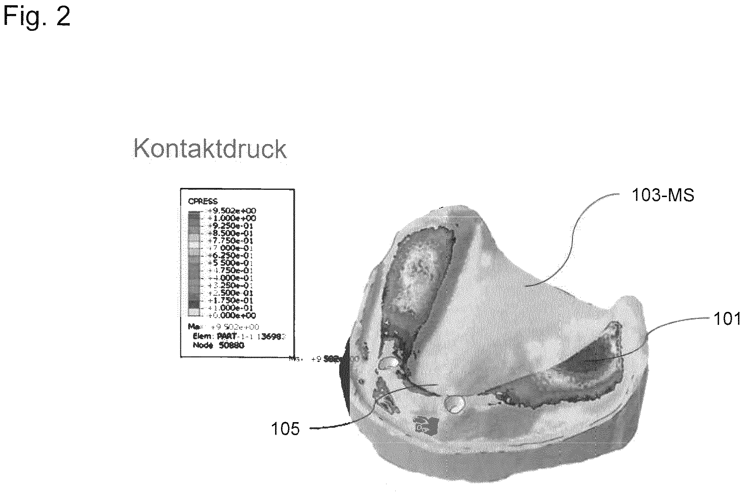

Fig. 2 zeigt eine Druckverteilung 101 auf der Mundschleimhaut 105 durch die dentale Restauration 100. Die Druckverteilung 101 wird aus dem dreidimensionalen Datensatz 103-DR der dentalen Restauration 100 und dem dreidimensionalen Datensatz 103-MS der Mundschleimhaut 105 berechnet. Zu diesem Zweck kann ein Finite-Elemente-Verfahren (FEM) durchgeführt werden, um die Druckverteilung 101 eines Kontakts der dentalen Restauration 100 auf der Mundschleimhaut 105 zu simulieren. Die Berechnungen berücksichtigen sowohl die mechanische Materialeigenschaften des Mundschleimhautes als auch der Prothese. Eine nicht lineare Material Modell kommt für die Beschreibung der mechanischen Eigenschaften des Mundschleimhautes im Einsatz. Andererseits kann das Materialverhalten von der Prothese Material als linearelastisch angenommen werden. Darüber hinaus muss für die Kontaktstelle zwischen Prothese und Mundschleimhaut geometrische nicht Linearitäten berücksichtigt werden. Durch die vollständige Beschreibung der mechanischen Eigenschaften von jeweiligen Materialen, können mit Hilfe von einem FEM basierten Kontaktalgorithmus die Druckverteilung berechnet werden. Auf der Mundschleimhaut 105 existieren Bereiche, in denen die dentale Restauration 100 einen geringen Druck auf die Mundschleimhaut 105 ausübt und Bereiche, in denen die dentale Restauration 100 einen hohen Druck auf die Mundschleimhaut 105 ausübt Bei der Simulation der Druckverteilung 101 kann die okklusale Oberfläche der dentalen Restauration 100 während eines Kauprozesses berücksichtigt werden. Hierzu können unterschiedliche Kauprozesse in Abhängigkeit einer Stelle und einer Art der Nahrung simuliert werden, um eine Druckverteilung 101 eines Kontaktdrucks der dentalen Restauration 100 auf der Mundschleimhaut 105 zu erhalten. Für die Optimierung der dentalen Restauration 100 kann ein Fall ausgewählt werden, bei dem die ungünstigsten Kräfte (Worst Case Scenario) auftreten. Dies führt zu einem maximalen Spitzenkontaktdruck auf die Mundschleimhaut 105.

Der simulierte lokale Spitzenkontaktdruck kann dann durch elastische Herstellungsmaterialien 109 der dentalen Restauration 100 minimiert werden, die ein geringes Elastizitätsmodul aufweisen als die Herstellungsmaterialien 113 der übrigen dentalen Restauration 100. Durch die Simulation kann eine Dicke des Herstellungsmaterials 109 mit dem niedrigeren Elastizitätsmodul optimiert werden, um einen Spitzenkontaktdruck unterhalb von einer Druckschmerzgrenze zu halten. Die Shore-Härte (Skala A) des Herstellungsmaterials 109 liegt beispielsweise in einem Bereich zwischen 35 und 60 (ISO 7619-1). Die Zugfestigkeit beträgt beispielsweise zwischen 5.5 und 8.5 N/mm² (ISO 37 Type 4).

Das elastische Herstellungsmaterial 109 für den räumlichen Bereich 107 ist beispielsweise ein Elastomer, wie Silikon. Das Herstellungsmaterial 113 für die dentale Restauration 100 ist beispielsweise ein ungefülltes oder gefülltes Methylmethacrylat (MMA) und weist typischerweise eine Härte auf im Bereich zwischen 41.63 ± 2.03 und 34.62 ± 2.1 auf (Vickers-Härte). Eine Biegefestigkeit (MPa) liegt beispielsweise zwischen 86.63 ± 1.0 und 69.15 ± 0.88. Eine Schlagfestigkeit (KJ/m²) liegt beispielsweise zwischen 6.32 ± 0.50 und 2.44 ± 0.31.

Das Herstellungsmaterial 109 für den für den räumlichen Bereich 107 ist weicher oder elastischer als das Herstellungsmaterial 113 für die dentale Restauration 100.

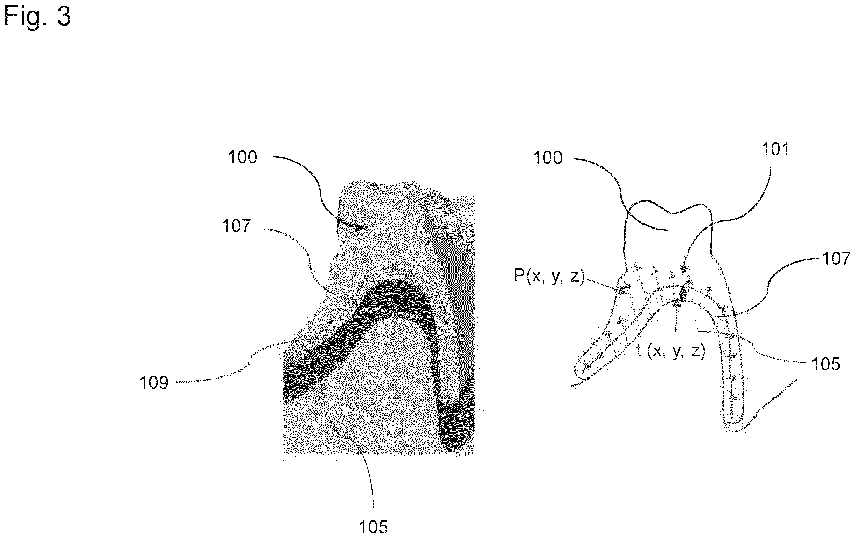

Fig. 3 zeigt eine Querschnittsansicht durch die dentale Restauration 100 und die Mundschleimhaut 105. Nach der der Definition von vorgegebenen Kräften auf die dentale Restauration 100 kann ein Optimierungsalgorithmus verwendet werden, der den Spitzenkontaktdruck der dentalen Restauration 100 unter die Druckschmerzgrenze des Patienten drückt. Dabei wird der dreidimensionale Datensatz 103-DR der dentalen Restauration 100 in Kombination mit dem dreidimensionalen Datensatz 103-MS der Mundschleimhaut 105 verwendet. Zunächst wird unter den vorgegebenen Kräften eine Druckverteilung 101 auf der Mundschleimhaut 105 berechnet.

Dabei wird eine Dicke *t(x,y,z)* eines räumlichen Bereichs 107 des elastischen Herstellungsmaterial 109 so optimiert, dass eine möglichst gleichförmige Druckverteilung *P(x,y,z)* auf die Mundschleimhaut 105 ausgeübt wird. Unter der Annahme einer anfänglichen monolithischen oder homogenen Materialverteilung in der Basis der dentalen Prothese 100 wird eine dreidimensionale Verteilung eines elastischen Herstellungsmaterials 109 durch die simulierte Druckverteilung *P(x,y,z)* auf die Mundschleimhaut 105 abgeleitet. Diese Verfahren kann mehrere Male iterativ wiederholt werden, beispielsweise solange bis ein Spitzenkontaktdruck der Druckverteilung 101 unter einem vorgegebenen Wert liegt.

Die Ausführung des Algorithmus kann auf einem Computer erfolgen, auf dem ein Programm zum Ausführen des Verfahrens gespeichert ist. Der Computer umfasst hierzu einen Prozessor, mit dem Befehle zur Ausführung des Algorithmus verarbeitet werden und einen digitalen Speicher, in dem die dreidimensionalen Datensätze 103 der Mundschleimhaut 105 und der dentalen Restauration 100 gespeichert sind.

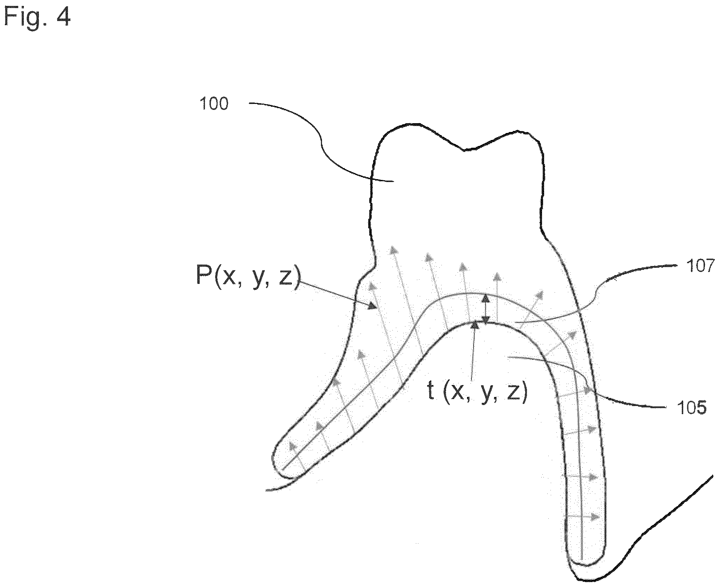

Fig. 4 zeigt eine weitere Querschnittsansicht durch die dentale Restauration 100 und die Mundschleimhaut 105. Die Druckverteilung 101 auf der Oberfläche wird durch Kraftvektoren je Flächeneinheit angegeben, bei der die Kraftvektoren von den Vertices ausgehen und im Idealfall senkrecht auf der Fläche oder dem Vertex stehen. Die lokale Dicke des elastischen Herstellungsmaterials 109 an der basalen Fläche der Prothesenbasis wird durch Anwenden eines konstanten Umrechnungsfaktors auf jeden Kraftvektor berechnet.

Auf diese Weise wird eine neue interne Fläche durch ein Definieren neuer Vertices am Ende jedes neuberechneten Vektors erzeugt. Auf diese Weise kann ein räumlicher Bereich 109 berechnet werden, dessen lokale Dicke von der Druckverteilung 111 der dentalen Restauration 100 abhängt. Zusammen mit der äußeren basalen Oberfläche, an der die Kraftvektoren an den Vertices anstehen, erzeugt die neu erzeugte interne Fläche ein Untervolumen der dentalen Restauration. In dem räumlichen Bereich 107 des Untervolumens wird das elastische Herstellungsmaterial 109 angeordnet. Die Form und die Position des räumlichen Bereichs 107 kann in den dreidimensionalen Datensatz 103-DR der dentalen Restauration 100 integriert werden.

Um eine Überschneidung oder Überlappung des ursprünglichen Basisvolumens und dem neu erzeugten räumlichen Bereich 109 für das elastische Herstellungsmaterial 109 der dentalen Restauration 100 zu vermeiden, wird eine Boolesche Operation angewendet, durch die der neu erzeugte räumliche Bereich von dem Volumen der ursprünglichen dentalen Restauration 100 abgezogen wird. In der Druck-CAM-Software werden die Materialtypen in Abhängigkeit ihres Elastizitätsmoduls zu den getrennten räumlichen Bereichen zugewiesen.

Im Allgemeinen kann die Berechnung des räumlichen Bereichs für das elastische Herstellungsmaterial 109 auch auf andere Weise berechnet werden. Beispielsweise kann auch sobald ein Wert der Druckverteilung 101 über einem vorgegebenen Wert liegt, ein räumlicher Bereich 107 mit einer vorgegebenen Dicke vorgesehen sein.

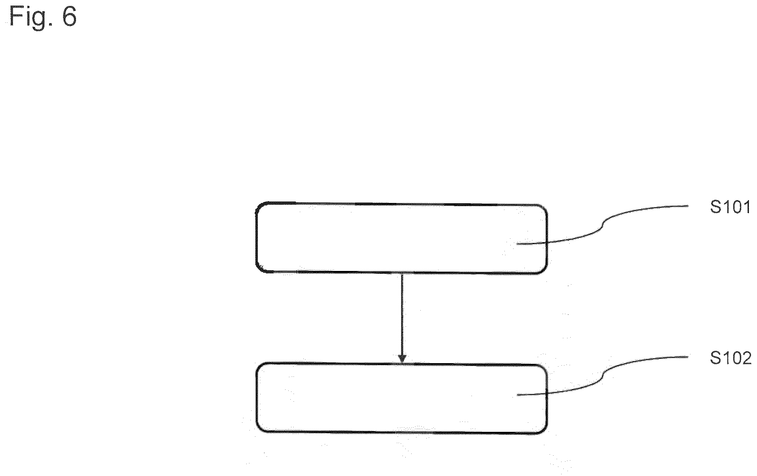

Fig. 6 zeigt ein Blockdiagramm eines Verfahrens zum Herstellen der dentalen Restauration 100. Im Schritt S101 wird die Druckverteilung 101 auf die Mundschleimhaut 105 durch die dentale Restauration 100 auf Basis eines dreidimensionalen Datensatzes 103-DS der dentalen Restauration 100 und eines dreidimensionalen Datensatzes 103-MS der Mundschleimhaut 105 berechnet. Anschließend wird in Schritt S102 ein räumlicher Bereich 107 für das elastische Herstellungsmaterial 109 innerhalb der dentalen Restauration 100 auf Basis der berechneten Druckverteilung 101 bestimmt. Anschließend wird die dentale Restauration 100 mit dem elastischen Herstellungsmaterial 109 hergestellt.

Um die unterschiedlichen Herstellungsmaterialien 109 an unterschiedlichen Stellen innerhalb des dreidimensionalen Modells anzuordnen, kann bevorzugt ein additives Fertigungsverfahren, bei dem ein selektiver Materialauftrag unterschiedlicher Materialien möglich ist (Extrusion, Freistrahl-Verfahren, Inkjet), verwendet werden, wobei die unterschiedliche Materialien verschiedene Elastizitätsmodule aufweisen.

Das harte und feste Herstellungsmaterial der dentalen Restauration 100 kann beispielsweise Methylmethacrylat (MMA) sein und das elastische Herstellungsmaterial 109 kann ein lichthärtbares medizinisches Silikon sein. Durch ein hartes und ein weiches herstellungsmaterial innerhalb des Systems, kann auch ein dazwischenliegendes Elastizitätsmodul durch Mischen der beiden Herstellungsmaterialien in einem bestimmten Verhältnis erreicht werden.

Durch das Verfahren wird der Tragekomfort der dentalen Restauration unter Berücksichtigung der individuellen Anatomie eines Patienten erhöht. Ein Schmerz oder Beschwerden aufgrund inhomogener Druckverteilungen an der dentalen Restauration 100 kann minimiert werden. Eine langfristige Restkammresorption kann verhindert werden. Zudem wird ein besserer Langzeittragekomfort aufgrund des elastischen Materials in Kontakt mit der Mundschleimhaut erreicht werden, das eine anatomische Entwicklung der Mundschleimhaut effektiv zulässt.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentale Restauration
- 101: Druckverteilung
- 103: Datensatz
- 105: Mundschleimhaut
- 107: räumlicher Bereich
- 109: Herstellungsmaterial
- 111: Druckverteilung
- 113: Herstellungsmaterial

## Patentansprüche

1. Verfahren zum Herstellen einer dentalen Restauration (100), mit den Schritten:
- Berechnen (S101) einer Druckverteilung (101) auf die Mundschleimhaut (105) durch die dentale Restauration (100) auf Basis eines dreidimensionalen Datensatzes (103-DS) der dentalen Restauration (100) und eines dreidimensionalen Datensatzes (103-MS) der Mundschleimhaut (105); und
- Bestimmen (S102) eines räumlichen Bereiches (107) für ein elastisches Herstellungsmaterial (109) innerhalb der dentalen Restauration (100) auf Basis der berechneten Druckverteilung (101).

2. Verfahren nach Anspruch 1, wobei ein Herstellungsmaterial für den räumlichen Bereich (107) auf Basis der Druckverteilung (101) bestimmt wird.

3. Verfahren nach einem der vorangehenden Ansprüche, wobei die Druckverteilung (101) mittels einer Finite-Elemente-Methode berechnet wird.

4. Verfahren nach einem der vorangehenden Ansprüche, wobei die Druckverteilung (101) bei Vorliegen einer vorgegebenen Kraft auf die dentale Restauration (100) berechnet wird.

5. Verfahren nach Anspruch 4, wobei die vorgegebene Kraft durch einen Aufbiss-Sensor ermittelt wird.

6. Verfahren nach einem der vorangehenden Ansprüche, wobei aus der Druckverteilung (101) auf die Mundschleimhaut (105) eine Druckverteilung (111) auf die dentale Restauration (100) berechnet wird.

7. Verfahren nach Anspruch 6, wobei eine Dicke des räumlichen Bereichs (107) proportional zur Druckverteilung (111) auf die dentale Restauration (100) ist.

8. Verfahren nach einem der vorangehenden Ansprüche, wobei das Herstellungsmaterial (109) in dem räumlichen Bereich (107) ein niedrigeres Elastizitätsmodul als das Herstellungsmaterial (113) außerhalb des räumlichen Bereichs (107) aufweist.

9. Verfahren nach einem der vorangehenden Ansprüche, wobei das Herstellen der dentalen Restauration (100) mittels eines dreidimensionalen Druckverfahrens durchgeführt wird.

10. Verfahren nach Anspruch 9, wobei das dreidimensionale Druckverfahren einen Freistrahl-Materialauftrag verwendet.

11. Herstellungsvorrichtung (200) zum Herstellen einer dentalen Restauration (100), mit:
- einer Berechnungsvorrichtung (201) zum Berechnen einer Druckverteilung (101) auf die Mundschleimhaut (105) durch die dentale Restauration (100) auf Basis eines dreidimensionalen Datensatzes (103-DS) der dentalen Restauration (100) und eines dreidimensionalen Datensatzes (103-MS) der Mundschleimhaut (105); und
- einer Bestimmungsvorrichtung (203) zum Bestimmen eines räumlichen Bereiches (107) für ein elastisches Herstellungsmaterial (109) innerhalb der dentalen Restauration (100) auf Basis des bestimmten Druckverteilung (101).

12. Herstellungsvorrichtung (200) nach Anspruch 11, wobei die Berechnungsvorrichtung (201) ausgebildet ist, die Druckverteilung (101) mittels einer Finite-Elemente-Methode zu berechnen.

13. Herstellungsvorrichtung (200) nach einem der Ansprüche 11 oder 12, wobei die Bestimmungsvorrichtung (203) ausgebildet ist, eine Dicke des räumlichen Bereichs (107) proportional zur Druckverteilung (111) auf eine dentale Restauration (100) zu bestimmen.

14. Herstellungsvorrichtung (200) nach einem der Ansprüche 10 bis 13, wobei Herstellungsvorrichtung (200) ausgebildet ist, innerhalb des räumlichen Bereiches (107) ein Herstellungsmaterial (109) mit einem niedrigeren Elastizitätsmodul als außerhalb des räumlichen Bereichs (107) zu verwenden.

15. Herstellungsvorrichtung (200) nach einem der Ansprüche 11 bis 14, wobei die Herstellungsvorrichtung (200) einen 3D-Drucker umfasst.

## Claims

1. A method of producing a dental restoration (100), comprising the steps of:
- calculating (S101) a pressure distribution (101) on the oral mucosa (105) through the dental restoration (100) based on a three-dimensional data set (103-DS) of the dental restoration (100) and a three-dimensional data set (103-MS) of the oral mucosa (105); and
- determining (S102) a spatial region (107) for an elastic production material (109) within the dental restoration (100) based on the calculated pressure distribution (101).

2. The method according to claim **1,** wherein a production material for the spatial region (107) is determined based on the pressure distribution (101).

3. The method according to any of the preceding claims,
wherein the pressure distribution (101) is calculated by means of a finite element method.

4. The method according to any of the preceding claims,
wherein the pressure distribution (101) is calculated in the presence of a predetermined force on the dental restoration (100).

5. The method according to claim 4, wherein the predetermined force is determined by a bite block sensor.

6. The method according to any of the preceding claims, wherein a pressure distribution (111) on the dental restoration (100) is calculated from the pressure distribution (101) on the oral mucosa (105).

7. The method according to claim 6, wherein a thickness of the spatial region (107) is proportional to the pressure distribution (111) on the dental restoration (100).

8. The method according to any of the preceding claims,
wherein the production material (109) in the spatial region (107) has a lower modulus of elasticity than the production material (113) outside the spatial region (107) .

9. The method according to any of the preceding claims,
wherein the production of the dental restoration (100) is carried out by means of a three-dimensional printing process.

10. The method according to claim 9, wherein the three-dimensional printing process uses a free-jet material deposition.

11. A production device (200) for producing a dental restoration (100), comprising:
- a calculation device (201) for calculating a pressure distribution (101) on the oral mucosa (105) through the dental restoration (100) based on a three-dimensional data set (103-DS) of the dental restoration (100) and a three-dimensional data set (103-MS) of the oral mucosa (105); and
- a determination device (203) for determining a spatial region (107) for an elastic production material (109) within the dental restoration (100) based on the determined pressure distribution (101).

12. The production device (200) according to claim 11, wherein the calculation device (201) is configured to calculate the pressure distribution (101) by means of a finite element method.

13. The production device (200) according to any of claims 11 or 12, wherein the determination device (203) is configured to determine a thickness of the spatial region (107) proportional to the pressure distribution (111) on a dental restoration (100).

14. The production device (200) according to any of claims 10 to 13, wherein the production device (200) is configured to use a production material (109) having a lower modulus of elasticity within the spatial region (107) than outside the spatial region (107).

15. The production device (200) according to any of claims 11 to 14, wherein the production device (200) comprises a 3D printer.

## Revendications

1. Procédé de réalisation d'une restauration dentaire (100), comportant les étapes suivantes :
- calcul (S101) d'une distribution de pression (101) sur la muqueuse buccale (105) par la restauration dentaire (100) sur la base d'un ensemble de données tridimensionnelles (103-DS) de restauration dentaire (100) et d'un ensemble de données tridimensionnelles (103-MS) de la muqueuse buccale (105) ; et
- détermination (S102) d'une région spatiale (107) pour un matériau de fabrication élastique (109) à l'intérieur de la restauration dentaire (100) sur la base de la distribution de pression calculée (101).

2. Procédé selon la revendication 1, où un matériau de fabrication pour la région spatiale (107) est déterminé sur la base de la distribution de pression (101).

3. Procédé selon l'une des revendications précédentes, où la distribution de la pression (101) est calculée au moyen d'une méthode des éléments finis.

4. Procédé selon l'une des revendications précédentes, où la distribution de la pression (101) est calculée en présence d'une force donnée sur la restauration dentaire (100).

5. Procédé selon la revendication 4, où la force spécifiée est déterminée par un capteur occlusal.

6. Procédé selon l'une des revendications précédentes, où une distribution de pression (111) sur la restauration dentaire (100) est calculée à partir de la distribution de pression (101) sur la muqueuse buccale (105).

7. Procédé selon la revendication 6, où une épaisseur de la région spatiale (107) est proportionnelle à la répartition de la pression (111) sur la restauration dentaire (100).

8. Procédé selon l'une des revendications précédentes, où le matériau de fabrication (109) dans la région spatiale (107) présente un module d'élasticité inférieur à celui du matériau de fabrication (113) en dehors de la région spatiale (107).

9. Procédé selon l'une des revendications précédentes, où la préparation de la restauration dentaire (100) est réalisée au moyen d'un procédé d'impression tridimensionnelle.

10. Procédé selon la revendication 9, où le procédé d'impression tridimensionnelle utilise une application de matériau à jet libre.

11. Dispositif de fabrication (200) pour la réalisation d'une restauration dentaire (100), comprenant :
- un dispositif de calcul (201) pour calculer une répartition de pression (101) sur la muqueuse buccale (105) par la restauration dentaire (100) sur la base d'un ensemble de données tridimensionnelles (103-DS) de la restauration dentaire (100) et d'un ensemble de données tridimensionnelles (103-MS) de la muqueuse buccale (105) ; et
- un dispositif de détermination (203) permettant de déterminer une région spatiale (107) pour un matériau de fabrication élastique (109) à l'intérieur de la restauration dentaire (100) sur la base de la distribution de pression déterminée (101).

12. Dispositif de fabrication (200) selon la revendication 11, où l'appareil de calcul (201) est conçu pour calculer la distribution de pression (101) au moyen d'une méthode des éléments finis.

13. Dispositif de fabrication (200) selon l'une des revendications 11 ou 12, où le dispositif de détermination (203) est conçu pour déterminer une épaisseur de la région spatiale (107) proportionnelle à la répartition de la pression (111) sur une restauration dentaire (100).

14. Appareil de fabrication (200) selon l'une des revendications 10 à 13, où l'appareil de fabrication (200) est conçu pour utiliser un matériau de fabrication (109) avec un module d'élasticité plus faible à l'intérieur de la région spatiale (107) qu'en dehors de la région spatiale (107).

15. Dispositif de fabrication (200) selon l'une des revendications 11 à 14, où le dispositif de fabrication (200) comprend une imprimante 3D.
